Europäisches Patentamt

European Patent Office

Office européen des brevets

(19)

(11) Publication number: **0 246 090**
**A2**

## (12) EUROPEAN PATENT APPLICATION

(21) Application number: **87304259.2**

(22) Date of filing: **13.05.87**

(51) Int. Cl.⁴: **A 61 K 7/06**

(30) Priority: **15.05.86 JP 109620/86**

(43) Date of publication of application:
**19.11.87 Bulletin 87/47**

(84) Designated Contracting States:
**AT BE CH DE ES FR GB IT LI NL SE**

(71) Applicant: **LION CORPORATION**
**No. 3-7, 1-chome Honjyo**
**Sumida-ku Tokyo (JP)**

(72) Inventor: **Iwao, Shuji**
**17-33, Kasuga-cho 4-chome§Nerima-ku**
**Tokyo (JP)**

**Kuwana, Hiroko**
**Bira Kobayashi 2-B 13-13, Maeharanishi 5-chome**
**Funabashi-shi Chiba (JP)**

(74) Representative: **Fisher, Bernard et al**
**Raworth, Moss & Cook 36 Sydenham Road**
**Croydon Surrey CR0 2EF (GB)**

(54) **Hair cosmetic composition.**

(57) A hair cosmetic composition comprising a cationic polymeric material and a cyclodextrin. This hair cosmetic composition has an excellent perm odor inhibition effect and a high persistence of the inhibition effect.

EP 0 246 090 A2

**Description**

## HAIR COSMETIC COMPOSITION

### BACKGROUND OF THE INVENTION

1. Field of the Invention

The present invention relates to a hair cosmetic composition having an extremely good perm odor inhibition effect after a permanent wave treatment, which inhibiting effect persists for a long time.

2. Description of the Related Art

An unpleasant odor is generated after a permanent wave treatment composed mainly of mercaptan, and a hair cosmetic composition is needed which will inhibit the perm odor and continue the inhibition effect for a long time, but in the present state of the prior art, a satisfactorily effective proposal for inhibiting perm odor has not been made.

It is known in the prior art that a cyclodextrin used as the microcapsule substance encapsulating, for example, perfumes, taste substances, or pharmaceuticals, has a deodorizing effect. For example, Japanese Unexamined Patent Publication (Kokai) No. 53-41440 discloses that a cyclodextrin has the effect of deodorizing objectionable odors such as axillary odor, foot odor, or mouth odor, and that a deodorizing agent harmless to human and animals without secondary drawbacks and without application limitations can be obtained by formulation of a cyclodextrin. Also, for application to the hair, for example, Japanese Unexamined Patent Publications (Kokai) Nos. 59-84809 and 59-84810 disclose formulation of a compound such as p-nitroacetophenone, or methyl-$\beta$-naphthyl ketone, which together with a cyclodextrin, is formulated into the first agent of a permanent wave treatment mainly composed of a mercapto compound, whereby the mercaptan odor inherent in the first agent is inhibited or a large amount of the mercaptan odor generated when the first agent is applied to hair is inhibited.

However, the deodorizing effect of cyclodextrin against perm odor is not completely satisfactory, and is particularly inferior in the persistence of the effect, and it has been impossible to fully prevent the generation of odor from the head portion even if a cyclodextrin is formulated in the hair cosmetic.

It also has been proposed to inhibit perm odor with abietic acid or its derivative, but this effect proved to be unsatisfactory.

### SUMMARY OF THE INVENTION

Accordingly, the objects of the present invention are to eliminate the above-mentioned disadvantages of the prior art and to provide a hair cosmetic composition having an excellent perm odor inhibition effect and having a high persistence of the inhibition effect.

Other objects and advantages of the present invention will be apparent from the following description.

In accordance with the present invention, there is provided a hair cosmetic composition comprising a cationic polymeric material and a cyclodextrin.

### DESCRIPTION OF THE PREFERRED EMBODIMENTS

The present inventors have made intensive studies in order to accomplish the above object, and consequently found that, when a cationic polymeric material and a cyclodextrin are used in combination, both act synergetically to give a sufficient deodorizing effect against perm odor, and that this deodorizing effect persists for a long time, to accomplish the present invention.

The first essential component of the hair cosmetic composition of the present invention is a cationic polymeric material. The cationic polymeric material is not limited in kind, but particularly preferably, one or more of cationized cellulose, quaternarized vinylpyrrolidone-dimethylaminoethylmethacrylic acid copolymer, dimethyldiallylammonium chloride copolymer and dimethyldiallylammonium chloride-acryloamide copolymer, cationized chitin and chitosan, are used above all cationized cellulose.

In the hair cosmetic composition of the present invention, the amount of the cationic polymeric material formulated is preferably 0.01% to 7% by weight, particularly 0.1% to 3% by weight based on the total amount of the cosmetic composition. With an amount less than 0.01% by weight, the persistence of the perm odor inhibiting effect is insufficient, and an amount in excess of 7% by weight may cause stickiness in the hair.

The second essential component of the present hair cosmetic composition is a cyclodextrin, and as the cyclodextrin, one or more of cyclodextrins selected from $\alpha$-cyclodextrin, $\beta$-cyclodextrin, $\gamma$-cyclodextrin, $\delta$-cyclodextrin and cationized cyclodextrin are preferably employed, but most preferable is $\alpha$-cyclodextrin with respect to solubility in the system and the perm odor inhibiting effect. In this case, the amount of the cyclodextrin formulated is preferably 0.001% to 5% by weight, particularly 0.05% to 2% by weight, based on the total amount of the cosmetic. With an amount less than 0.001% by weight, the perm odor inhibiting effect is unsatisfactory, and an amount in excess of 5% by weight causes problems in the solubility in the system, and in the cost.

The proportion of the cationic polymeric material and the cyclodextrin to be used in combination is preferably 1:100 to 100:1, particularly 1:20 to 20:1, in terms of weight ratio. Also, the total amount of the cationic polymeric material and the cyclodextrin formulated is 0.01% to 12% by weight, particularly 0.15% to 5% by weight.

The hair cosmetic composition of the present invention can be prepared and applied as the first agent for a permanent wave treatment, shampoo, hair liquid, hair tonic, foamy hair-care material, and the like, but the hair cosmetic composition of the present invention can formulate suitably optional conventional components in addition to the above components, depending upon the kind of the cosmetic composition, including, for example, oil components such as silicon derivatives, hydrocarbons, ester oils, higher fatty acids, high alcohols, lanolin derivatives; nonionic surfactants, anionic surfactants, amphoteric surfactants, vitamin A, $B_6$, $B_{12}$, C, D, E, H and derivatives thereof, protein hydrolysates, amino acids, animal and vegetable extracts, nonionic resins such as polyvinylpyrrolidone and derivatives thereof; anionic resins such as methylvinyl ether-butyl maleate copolymer; amphoteric resins such as acrylic acid-methacrylate copolymers; UV-ray absorbers such as oxybenzoin derivatives, cinnamic acid ester derivatives; coolness imparting agents such as $\ell$-menthol, peppermint oil; stimulant feel imparting agents such as benzyl nicotinate, Guinea pepper tincture; sterilizers such as gluconic acid chlorohexidine, isopropylmethylphenol; preservatives such as paraben; perfumes, dyes, and deterioration preventives.

The hair cosmetic composition of the present invention is provided with an excellent perm odor inhibiting effect and a high persistence of this effect, by a combined use of a cationic polymer and a cyclodextrin.

EXAMPLES

The present invention will now be further described in more detail by referring to, but is by no means limited to, Examples and Comparative Examples.

Example 1 and Comparative Examples 1 and 2

First agents for a permanent wave treatment of Example 1 and Comparative Examples 1, 2 having the compositions shown in Table 1 were prepared, and the effects of perm odor inhibition and the persistence thereof possessed by these first agents were evaluated organoleptically by a panel of 20 members based on the following standards. The results are shown in Table 1. The amounts formulated in the Table are % by weight.

Evaluation Standards

◎ :  judged as good by 80% or more of panellers

o :  judged as good by 60 to less than 80% of panellers

Δ :  judged as good by 40 to less than 60% of panellers

x :  judged as good by less than 40% of panellers

Table 1

| Component | Comparative Example | | Example 1 |
|---|---|---|---|
| | 1 | 2 | |
| Cationized cellulose | 1.0 | – | 0.5 |
| α-Cyclodextrin | – | 1.0 | 0.5 |
| Ammonium thioglycolate (28%) | 13 | 13 | 13 |
| Monoethanolamine | 0.5 | 0.5 | 0.5 |
| Ammonium chloride | 2.5 | 2.5 | 2.5 |
| Tetrasodium ethylenediamine | 0.2 | 0.2 | 0.2 |
| Purified water | Balance | Balance | Balance |
| Total | 100.0 | 100.0 | 100.0 |
| Perm odor inhibiting effect | x | o | ◎ |
| Persistence of perm odor inhibiting effect | x | Δ | ◎ |

From the results in Table 1, the first agent for a permanent wave treatment of the Example in which a cationic polymeric material and a cyclodextrin are used in combination was found to have an excellent perm odor inhibiting effect and also persistence.

In contrast, when only a cationic polymeric material was used or when only a cyclodextrin was used, a satisfactory perm odor inhibiting effect and also persistence could not be obtained.

### Example 2: Shampoo

| Ingredient | % by weight |
|---|---|
| Sodium α-olefin sulfonate | 9.0 |
| Sodium polyoxyethylene lauryl ether sulfate | 6.0 |
| Coconut fatty acid diethanolamide | 3.0 |
| Cationized cellulose | 0.5 |
| Cationized cyclodextrin* | 0.2 |
| Dye | Adequate |
| Perfume | 0.3 |
| Purified water | Balance |
| Total | 100.0 |

    *    Reaction product of cyclodextrin and glycidyltrimethylammonium chloride

After the permanent treatment, the hair was washed with the above shampoo and the perm odor was evaluated. As the result, the perm odor was inhibited and the persistence of this effect was good.

### Example 3: Hair liquid

| Ingredient | % by weight |
|---|---|
| Polyoxypropylene (20) glyceryl ether | 15 |
| Dimethyldiallylammonium chloride copolymer | 0.3 |
| β-Cyclodextrin | 0.05 |
| Gluconic acid chlorohexidine | 0.05 |
| Vitamin derivative | 0.1 |
| Polyether modified silicone | 0.3 |
| Dye | Adequate |
| UV-ray absorber | 0.1 |
| Perfume | 0.3 |
| Ethanol | 30.0 |
| Purified water | Balance |
| Total | 100.0 |

The above hair liquid was applied on the hair after a permanent wave treatment and the perm odor was evaluated. As the result, the perm odor was inhibited, and the persistence of this effect was good.

### Example 4: Hair tonic

| Ingredient | % by weight |
|---|---|
| Benzyl nicotinate | 0.003 |
| Pantotenylethyl ether | 0.1 |
| ℓ-Menthol | 0.3 |
| Betadecanoic acid | 1.0 |
| Polyoxyethylene (40) hydrogenated castor oil | 1.0 |
| Vitamin E | 0.4 |
| Cationized cellulose | 0.1 |
| Cationized chitin | 0.1 |
| γ-Cyclodextin | 0.03 |
| Isopropylmethylphenol | 0.05 |
| Perfume | 0.3 |
| Dye | Adequate |
| Ethanol | 70 |
| Purified water | Balance |
| Total | 100.0 |

The above hair tonic was applied on the hair after a permanent treatment, and the perm odor was evaluated. As a result, the perm odor was inhibited, and the persistence of this effect was good.

### Example 5: Foamy hair-care material

| Ingredient | % by weight |
|---|---|
| Quaternarized vinylpyrrolidone-dimethylaminoethylmethacrylic acid copolymer | 1.5 |
| Sodium N-coconut fatty acid acyl-L-glutamate | 0.3 |
| Coconut fatty acid diethanolamide | 1.0 |
| Polyoxyethylene oleyl ether (15 E.O) | 0.5 |
| Keratin hydrolysate | 0.3 |
| Paraben | 0.1 |
| α-Cyclodextrin | 1.0 |
| Ethanol | 20.0 |
| Purified water | Balance |
| Total | 100.0 |

A foamy hair-care material was prepared by adding 4 parts by weight of a liquified petroleum gas (propellant) to 96 parts by weight of the base with the above composition.)

The above foamy hair-care material was applied on the hair after a permanent treatment, and the perm odor

6

was evaluated. As the result, the perm odor was inhibited, and the persistence of this effect was good.

## Claims

1. A hair cosmetic composition comprising a cationic polymeric material and a cyclodextrin.

2. A hair cosmetic composition as claimed in claim 1, wherein the cationic polymeric material is at least one member selected from the group consisting of cationized cellulose, quaternarized vinylpyrrolidone-dimethylaminoethylmethacrylic acid copolymer, dimethyldiallylammonium chloride copolymer, dimethyldiallylammonium chloride-acryloamide copolymer, cationized chitin, and cationized chitosan.

3. A hair cosmetic composition as claimed in claim 1, wherein the cyclodextrin is at least one member selected from the group consisting of α-cyclodextrin, β-cyclodextrin, γ-cyclodextrin, δ-cyclodextrin, and cationized cyclodextrin.

4. A hair cosmetic composition as claimed in claim 1, wherein the amount of the cationic polymeric material formulated is 0.01 to 7 wt.% based on the total amount.

5. A hair cosmetic composition as claimed in claim 1, wherein the amount of the cyclodextrin formulated is 0.001% to 5% by weight based on the total amount.